# EUROPEAN PATENT SPECIFICATION

(11) **EP 4 161 455 B1**
(45) Date of publication and mention of the grant of the patent: **25.11.2026**
(21) Application number: 21733512.4
(22) Date of filing: 02.06.2021
(51) Int. Cl.: A61F 5/44, A61F 5/445

(54) **AN OSTOMY APPLIANCE**
OSTOMIEVORRICHTUNG
ACCESSOIRE POUR STOMIE

(30) Priority: 05.06.2020 GB 202008524
(43) Date of publication of application: 12.04.2023
(73) Proprietor: Salts Healthcare Limited, Birmingham, West Midlands B7 4AA (GB)
(72) Inventor: POWNER, Iain, Birmingham, West Midlands B7 4AA (GB); ALFARO, Jesus, Birmingham, West Midlands B7 4AA (GB); HOWARD, Lee, Birmingham, West Midlands B7 4AA (GB)
(74) Representative: Forresters IP LLP
(86) International application number: PCT/GB2021/051361
(87) International publication number: WO 2021/245412

(56) References cited:
- EP-A2- 0 737 456
- EP-A2- 0 940 130
- WO-A1-02/13737
- WO-A1-2005/117775
- DE-A1- 102014 104 737
- US-A- 3 481 336
- US-A1- 2005 131 360

## Description

### Background

Ostomy appliances are well known. Example prior art ostomy appliances are shown in US2005/0131360 and WO2005/117775.

US2005/0131360 is considered the closest prior art to the invention and describes a drainable ostomy pouch comprising a first or proximal and a second or distal side wall of flexible sheet material sealed to each other for defining a cavity therebetween for receiving human stomal discharge through an aperture in the proximal side wall and an elongate drainage portion extending downwardly in a longitudinal direction and ending in a drainage opening extending transversely to the longitudinal direction. A first or proximal and a second or distal flexible stiffening strip of relatively stiff material such as PET, nylon, high density polyethylene or low density polyethylene extending immediately adjacent to and along said drainage opening and being attached for instance by heat sealing or adhesion to the outer surface of the first and second side walls, respectively, of said drainage portion. The pouch further comprises a Velcro strip on the wall and on a flap for securing a coil comprising the first and second stiffening strips rotated upwards three times.

Typically ostomy appliances have first and second walls which are connected at their peripheries to form a cavity for the collection of waste. The first or second wall is provided with a stoma-receiving opening for receipt of a user's stoma. Sone ostomy appliances may be supplied as a drainable ostomy appliance. This might be for use with an ileostomy, for example. A drainable ostomy appliance is typically provided with an outlet, formed from a wall, which is fluidly connected to the cavity to enable waste to be removed from the cavity. The outlet may have an opening to enable the removal of waste. The opening is typically flexible and movable between closed and open positions. A common issue with such ostomy appliances is that the outlet wall can become stuck to itself which results in it no longer being possible to empty the ostomy appliance. This may be caused by a vacuum within the ostomy appliance causing the outlet wall to stick to itself, static adhesion of the outlet wall to itself, or waste collected within the ostomy appliance causing the outlet wall to stick to itself.

The present invention seeks to address these problems.

According to a first aspect of the invention we provide an ostomy appliance including:
first and second walls connected to each other at or near their peripheries;
a stoma-receiving opening for receipt of a stoma;
a collecting cavity defined between the first and second walls wherein the stoma-receiving opening is fluidly connected to the collecting cavity;
a connection member connected to the first and/or second wall for attaching the appliance to a user or for attaching the appliance to a flange for attaching the appliance to a user;
an outlet which extends away from the stoma-receiving opening, the outlet including an outlet wall and terminating in an opening; and
a user graspable member for assisting in moving the outlet opening from a first, closed, condition to a second, open, condition;
wherein the user graspable member includes a graspable portion and an attachment portion, the attachment portion being positioned above the graspable portion and closer to the stoma-receiving opening than the graspable portion;
wherein the attachment portion is attached to the outlet wall.

The user graspable member may be positioned at or near the outlet opening.

The attachment portion may be positioned entirely above the graspable portion.

The graspable portion may be movable, e.g. pivotally movable, relative to the attachment portion.

The graspable portion may have a free end remote from the attachment portion which is moveable by a user away from the outlet wall.

The graspable portion may be attached to the attachment portion by a connection portion. The graspable portion may be pivotable about the connection portion.

The connection portion may define an axis A about which the graspable portion pivots.

The outlet may be foldable between an extended position and a stowed position. The attachment portion and graspable portion may lie in generally the same plane in the stowed position.

The ostomy appliance may include a stiffening member attached to or forming part of the outlet wall. The stiffening member may be positioned at or near the outlet opening.

The stiffening member may include a first edge and a second edge. The stiffening member second edge may be positioned closer to the stoma-receiving opening than the stiffening member first edge. A height Hs of the stiffening member may be defined between the first and second stiffening member edges. A periphery of the user graspable member may include a first edge and a second edge. The user graspable member second edge may be positioned closer to the stoma-receiving opening than the user graspable member first edge. A height Ht of the user graspable member may be defined between the first and second user graspable member edges. The height Ht may be less than the height Hs.

The user graspable member may lie entirely within a periphery of the stiffening member.

The ostomy appliance may include a security flap to secure the outlet in a stowed position.

The graspable portion may include a formation for receipt of a user's finger to permit a user to grasp the user graspable member.

The formation may form part of a periphery of the user graspable member.

The formation may be a recess of the periphery.

The recess may be curved or arcuate.

The recess may extend towards the attachment portion.

The formation may provide a different tactile or visual characteristic to a remainder of the graspable portion to indicate to a user which portion of the user graspable member to grasp.

The user graspable member may be formed of a synthetic material.

The user graspable member may be formed of polyester.

The outlet wall may be a continuation of or connected to one of the first or second walls which define the collecting cavity.

Embodiments of the invention will now be described by way of example only with reference to the accompanying drawings, of which:
Figure 1 is a front view of a first embodiment of an ostomy appliance in accordance with the present invention;
Figure 2 is a further front view of the ostomy appliance of figure 1;
Figure 3 is a further front view of the ostomy appliance of figure 1;
Figure 4 is a rear view of the ostomy appliance of figure 1;
Figure 5 is a further rear view of the ostomy appliance of figure 1;
Figure 6 is a further rear view of the ostomy appliance of figure 1;
Figure 7 is a cross-sectional view of the ostomy appliance of figure 1 along the plane P;
Figures 8 to 10 are cross-sectional views of the ostomy appliance of figure 1 along the plane P as the ostomy appliance is moved from an extended, in use, position to a folded, stowed, position;

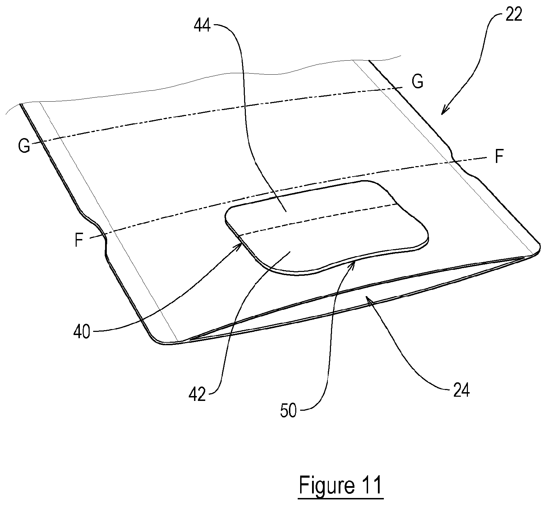

Figure 11 is an enlarged perspective view of the outlet of the ostomy appliance of figure 1;
Figure 12 is a front view of a second embodiment of an ostomy appliance in accordance with the present invention; and
Figure 13 is a front view of a third embodiment of an ostomy appliance in accordance with the present invention.

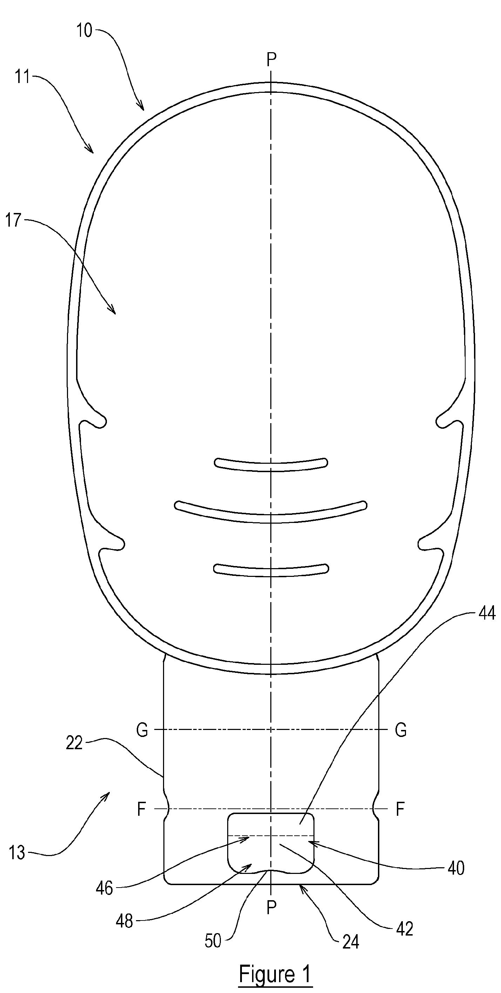

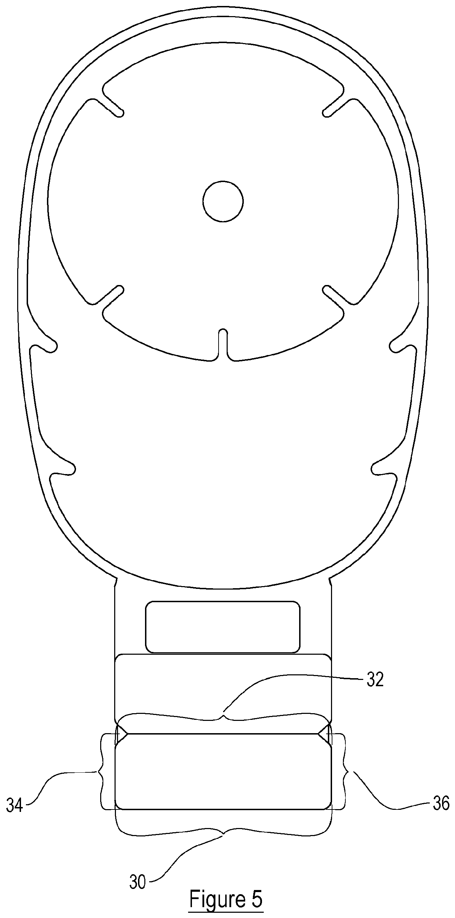

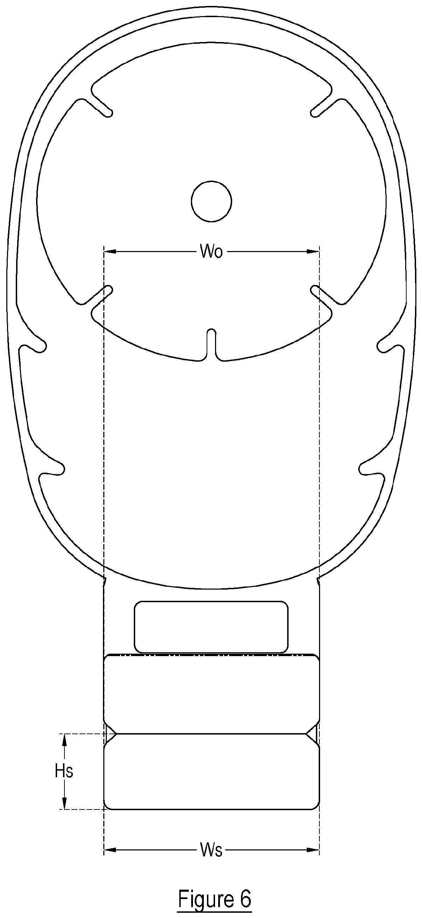

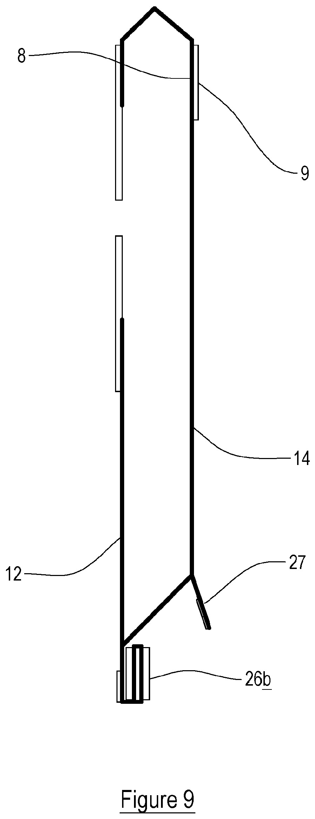

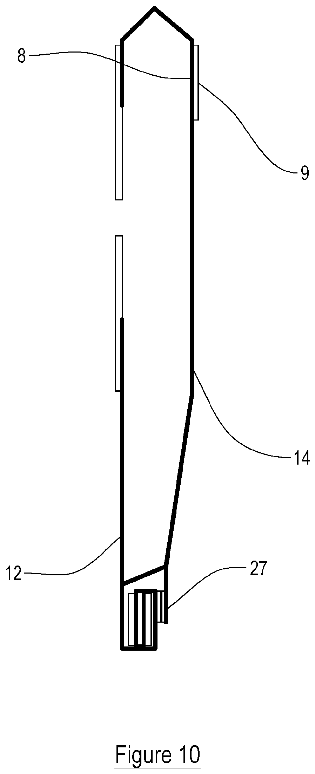

Referring to figures 1 to 10, these show a first embodiment of an ostomy appliance in accordance with the present invention, generally at 10. The ostomy appliance 10 has a top, or upper end, 11 and a bottom, or lower end, 13. The ostomy appliance 10 has first and second walls 12, 14 (preferably made from a plastics film material, which may be multi-layer) connected to each other at or near their peripheries, by any suitable means known in the art. For example, they could be adhered to each other or heat welded.

The first wall 12 includes a stoma-receiving opening 16 and supports a connection member or flange 20 for attaching the appliance 10 to a user. In the embodiment shown, the connection member 20 is a hydrocolloid wafer for securing the appliance 10 to the skin of a user around their stoma. Alternatively, the connection member 20 may be for attaching the appliance 10 to a flange for attaching the appliance to a user (e.g. a two-piece appliance as it is known in the art).

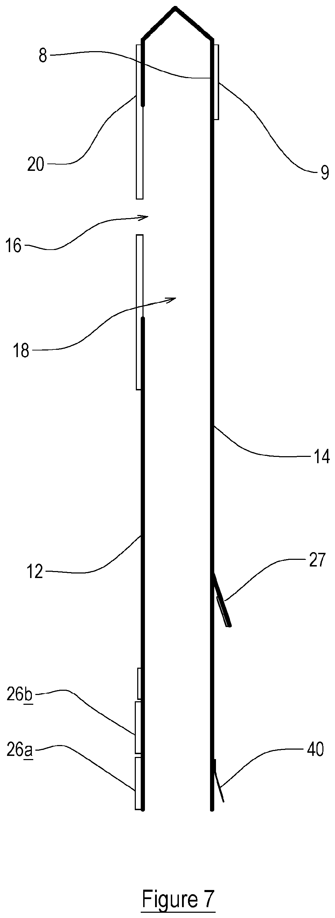

Referring to figure 7, the first and second walls 12, 14 define a waste collecting cavity 18 therebetween. In the present embodiment the first and second walls 12, 14 are covered by comfort layers 17 though these are not essential. For example only the first or second wall 12, 14 may be provided with a comfort layer, or neither the first nor the second wall 12, 14 may be provided with a comfort layer. The appliance 10 may also have a filter 9 positioned over a suitable gas vent 8 in either or both of the first 12 or second 14 walls, as is well known in the art.

The lower end 13 of the appliance 10 may be provided with an outlet 22 including an outlet wall. In the present embodiment the outlet 22 is formed by portions of the first and second walls 12, 14, again, as is well known in the art. **In** other words the outlet 22 is a continuation of one or both of the first and second walls 12, 14 which define the waste collecting cavity 18. **In** other embodiments the outlet 22 may be formed from a wall which is separate to the first and second walls 12, 14. Such an outlet 22 may be connected to one of the first and/or second walls which define the waste collecting cavity 18.

The outlet 22 extends downwardly away from the stoma-receiving opening 16 and terminates at an opening 24. The opening 24 permits waste collected in the collecting cavity 18 to be expelled from the appliance 10. The opening 24 is movable between a first, closed, condition and a second, open, condition. When the opening 24 is in the second, open, condition waste is permitted to move from the waste collecting cavity 18 through the outlet 22 and out of the opening 24 to exit the ostomy appliance 10.

To assist a user in moving the outlet opening from the first condition to the second condition the ostomy appliance 10 includes a user graspable member 40 which is positioned at or near the outlet opening 24 and is attached to the outlet wall 22 by an attachment portion 44. **In** the present embodiment the user graspable member 40, or more specifically the attachment portion 44, may be adhered to the outlet wall 22. **In** other embodiments other attachment means may be used, e.g. welding, to attach the user graspable member 40/attachment portion 44 to the outlet wall 22. **In** embodiments, the user graspable member 40 may be a tab.

The user graspable member 40 also includes a graspable portion 42 which is connected to the attachment portion 44 by a connection portion 46. The attachment portion 44 is positioned above the graspable portion 42, preferably entirely, and the connection portion 46 is positioned between the graspable portion 42 and the attachment portion 44. **In** other words the attachment portion is positioned closer to the stoma-receiving opening 16 than the graspable portion 42. Such a user graspable member 40 may be advantageous. For example, users may be able to more easily locate and grasp the graspable portion 42 of the user graspable member 40 than compared with the prior art. Therefore, users may find such a user graspable member 40 easier to use than the prior art when attempting to empty an ostomy appliance 10.

A user may operate the user graspable member 40 by grasping the graspable portion 42 and moving the graspable portion 42 in a direction away from the outlet 22 and a user. The graspable portion 42 is movable relative to the attachment portion 44. In particular, the graspable portion 42 has a free end 48 remote from the attachment portion 44 which is moveable by a user away from the outlet wall 22. In the present embodiment the graspable portion 42 is pivotable about the connection portion 46. The connection portion defines an axis A, in the present embodiment, about which the graspable portion 42 pivots. In other words, the graspable portion 42 is pivotally movable with respect to the attachment portion 44.

The graspable portion 42 may form a larger proportion of the surface area (when viewed from the front, e.g. as shown in figure 1) of the user graspable member 40 than the attachment portion 44. In other embodiments the attachment portion 44 may form a larger proportion of the surface area of the user graspable member 40 than the graspable portion 44. The attachment portion 44 may form at least: 10%; 20%; 30%; 40%; 50%; 60%; 70%; 80%; or 90% of the area of the user graspable member 40. In such embodiments, the graspable portion 42 may form, respectively, at least: 90%; 80%; 70%; 60%; 50%; 40%; 30%; 20%; or 10% of the area of the user graspable member 40.

A user graspable member 40 having one or more or all of these features may be advantageous. Users may find it easier to manipulate the graspable portion 42 of the user graspable member 40 compared with the prior art. For example, a user with limited dexterity and visibility may find it easier to locate the free end 48 of the graspable portion after the outlet 22 has been extended to an emptying position as the free end will be adjacent the opening 24 of the outlet and thus readily accessible to a user. In addition, having the free end 48 of the graspable portion 42 extend in a direction from the attachment portion 42 to the outlet opening 24 permits a user to easily use their fingers rather than their thumbs to manipulate the user graspable member 40. This may be a more comfortable movement for a user than using their thumbs if they have arthritis for example.

The graspable portion 42 of the user graspable member 40 includes a formation 50 for receipt of a user's finger to permit a user to grasp the user graspable member. In the present embodiment the formation 50 forms part of a periphery of the user graspable member 40, and in particular is a curved, or arcuate, recess. The formation 50 extends towards the attachment portion 44, in particular in a direction from the outlet opening 24 towards the attachment portion 44. The formation 50 may be positioned at the free end 48 of the graspable portion 42 and may be positioned remote to and/or generally opposite the attachment portion 44 and the connection portion 46. The formation 50 is spaced from the outlet opening 24.

In embodiments the formation 50 provides a different tactile or visual characteristic to a remainder of the graspable portion 42 to indicate to a user which portion of the user graspable member 40 to grasp. In other embodiments the formation 50 may not be curved or arcuate. For example, the formation 50 may be a recess of the periphery formed by linear portions. The formation 50 may also be provided at edges 56, 58 of the user graspable member 40. Indeed, a plurality of formations may be provided on the graspable portion 42.

The formation 50 may not form part of a periphery of the user graspable member but instead may be positioned on a surface of the graspable portion to indicate to a user which portion of the user graspable member 40 their hand is touching. For example the formation 50 may be a protrusion which extends away from, e.g. in a direction generally perpendicular to, a plane in which the graspable portion 42 lies and may extend either away from or towards a user.

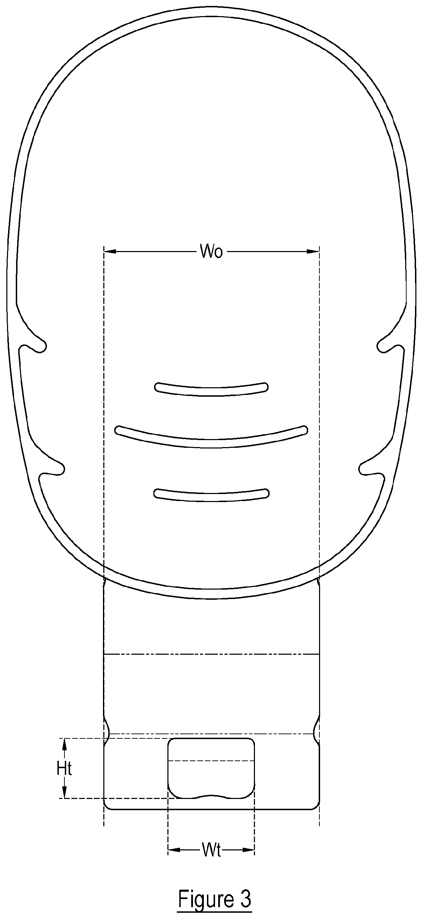

The user graspable member 40 extends laterally across the outlet 22 such that it extends generally perpendicularly to the direction in which waste exits the appliance 10, as shown in figures 1 to 3. The user graspable member 40 has a periphery including first and second generally opposite edges 52, 54 and third and fourth generally opposite edges 56, 58. The second edge 54 is positioned closer to the stoma-receiving opening than the first edge 52. The third and fourth edges 56, 58 are generally parallel with respect to each other. The first and second edges 52, 54 are connected to each other by the third and fourth edges 56, 58. Further curved portions may connect the first, second, third and fourth edges 52, 54, 56, 58 to each other. Referring to figure 3, a height Ht of the user graspable member 40 is defined between the first and second edges 52, 54. A width Wt of the user graspable member 40 is defined between the third and fourth edges 56, 58. The height Ht is less than the width Wt. In other embodiments the height Ht may be the same as or greater than the width Wt. The width Wt of the user graspable member 40 is less than a width Wo of the outlet 22, defined between laterally extending sides of the outlet 22, and/or the outlet opening 24. The user graspable member 40 is positioned generally centrally between the sides of the outlet 22.

The user graspable member 40 in the present embodiment has a thickness which is similar to that of the first and second walls 12, 14. Such a thickness of the user graspable member 40 does not materially affect the flexibility of the wall to which the user graspable member 40 is attached.

The user graspable member 40 is formed of a synthetic material. In the present embodiment the user graspable member 40 is formed of polyester. However, other materials could be used without departing from the scope of the present invention. For example, a textile, in particular a woven textile, may be used to form the user graspable member. It should be understood that the term textile encompasses woven, knitted and non-woven materials, as is well understood in the art.

In the present embodiment, first and second elongate stiffening members 26a, 26b are provided to assist with opening, closing and sealing of the opening 24. However, in other embodiments only a single stiffening member 26a may be provided. The stiffening member 26a may be positioned at or near the opening 24.

The first stiffening member 26a is connected, e.g. adhered or welded, to the exterior surface of the first wall 12, which faces the user and is positioned at or near the outlet opening 24. In alternative embodiments the first stiffening member 26a forms part of the outlet 22. The first stiffening member 26a extends laterally across the outlet 22 such that it extends generally perpendicularly to the direction in which waste exits the appliance 10, as shown in figure 3.

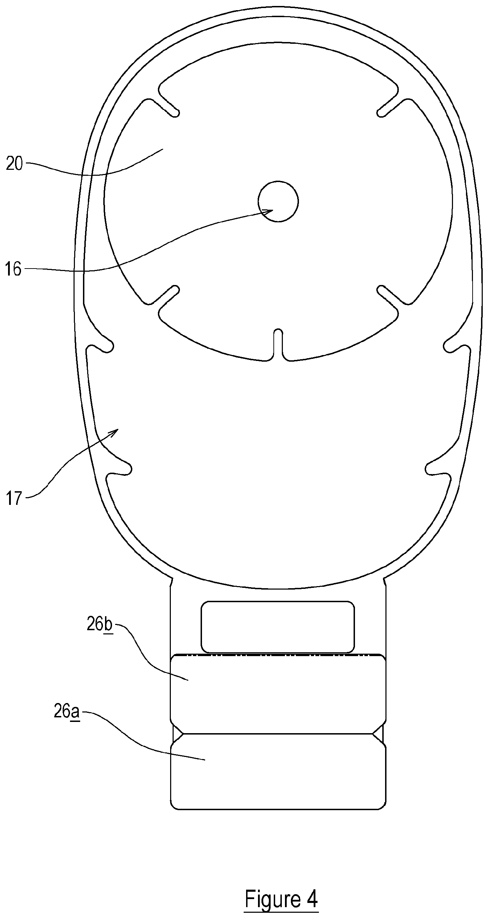

The second stiffening member 26b is positioned adjacent and above the first stiffening member 26a, further away from the opening 24 of the outlet 22 than the first stiffening member 26a, and is connected, e.g. adhered or welded, to the exterior surface of the first wall 12 (the same wall as the first stiffening member 26a). The second stiffening member 26b also extends laterally across the outlet 22 such that it extends generally perpendicularly to the direction in which waste exits the appliance 10, as shown in figures 4 to 6.

The stiffening members 26a, 26b need only be stiff in the sense that they are relatively stiff compared to the walls 12, 14 of the ostomy appliance 10. In this embodiment the stiffening members 26a, 26b are injection moulded from a plastics material. For example, the stiffening members 26a, 26b may be made from high impact polystyrene (HIPS).

The stiffening members 26a, 26b are substantially the same and so only one of the stiffening members 26a will be described in more detail. It should be appreciated however that in other embodiments the stiffening members 26a, 26b may be different to each other.

The stiffening member 26a has a periphery including first and second generally opposite edges 30, 32 and third and fourth generally opposite edges 34, 36. The second edge 32 is positioned closer to the stoma-receiving opening 16 than the first edge 30. The third and fourth edges 34, 36 are generally parallel with respect to each other. The first and second edges 30, 32 are connected to each other by the third and fourth edges 34, 36. Further curved portions may connect the first, second, third and fourth edges 30, 32, 34, 36 to each other. A height Hs of the stiffening member 26a is defined between the first and second edges 30, 32. A width Ws of the stiffening member 26a is defined between the third and fourth edges 34, 36. The height Hs is less than the width Ws. In other embodiments the height Hs may be the same as or greater than the width Ws. The width Ws of the stiffening member 26a is substantially that same as the width Wo of the outlet 22, defined between laterally extending sides of the outlet 22, and/or the outlet opening 24. In other embodiments the width Ws may be greater than the width Wo.

The height Ht of the user graspable member 40 is less than the height Hs of the stiffening member 26a. The width Wt of the user graspable member 40 is less than the width Ws of the stiffening member 26a. In particular, the user graspable member 40 lies entirely within the periphery of the stiffening member 26a. The user graspable member 40 is positioned generally centrally between the third and fourth edges 34, 36 of the stiffening member 26a. The user graspable member 40 is positioned generally closer to the second edge 32 of the stiffening member 26a than the first edge 30 of the stiffening member 26a.

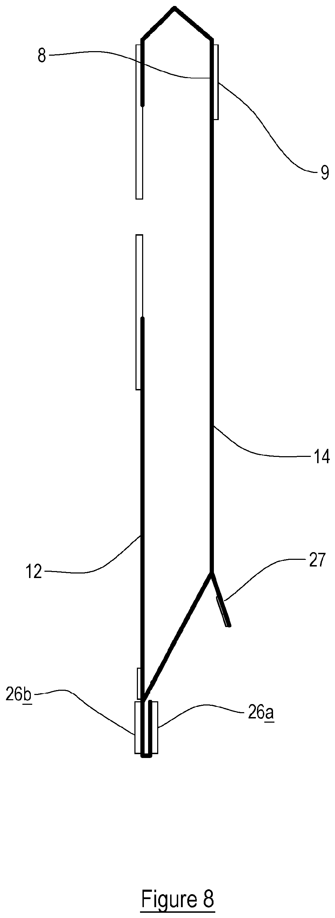

The outlet 22 is foldable, or rollable, between an extended position and a stowed position as shown by figures 7 to 10. The ostomy appliance 10 includes a security flap 27 (which may lie underneath the comfort layer 17) to secure the outlet in its stowed position. In the stowed position the attachment portion 44 and graspable portion 42 lie in generally the same plane, as shown in figures 8 to 10.

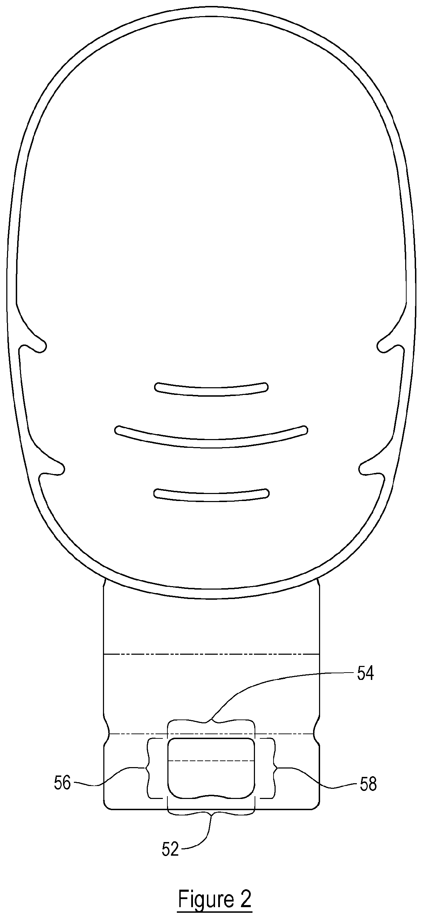

The outlet 22 may be stored, or stowed, by being rolled up around the stiffening members 26a, 26b, as is generally known in the art, and as shown in figures 7 to 10. Such rolling up uses the first and second edges 30, 32 of the members 26a, 26b as a fulcrum(s) to assist in tight closing of the outlet 22 and outlet opening 24, to prevent leaking. In particular, the stiffening members 26a, 26b define fold lines F-F and G-G as shown in figures 1 and 2. The user graspable member 40 is positioned below the fold line F-F. The user graspable member, and in particular the second edge 54 of the user graspable member 40, is positioned adjacent the fold line F-F.

In the present embodiment, the stiffening members 26a, 26b are rolled up two or three times, to seal the opening 24 in a closed condition. Any suitable fastening means, e.g. hook, and loop (Velcro) or adhesive, may be provided on respective faces of the flap 27 and outlet 22 to secure the outlet 22.

As explained above, the stiffening members 26a, 26b are substantially identical to each other and are configured such that when one member 26a is rotated 180 degrees and positioned on top of the other member 26b, they mate and cooperate with one another. Thus, the facing surfaces of the members 26a, 26b cooperate with each other so as to substantially close the outlet 22 therebetween.

To open the opening 24 of the appliance 10 the stiffening members 26a, 26b are bent by applying finger pressure to their third and fourth respective edges 34, 36, such that they bow. At the same time the graspable portion 42 is moved in a direction away from the outlet 22 and the user to move the opening 24 from the first, closed, condition to the second, open, condition. Waste is then permitted to move from the waste collecting cavity 18 through the outlet 22 and out of the opening 24 to exit the ostomy appliance 10. Once finger pressure is removed from the stiffening members 26a, 26b and the user releases the graspable portion 42, the opening 24 moves to or substantially moves to its first, closed, condition due to the stiffening members 26 being biased to a relatively flat position.

A second exemplary embodiment of the present invention will now be described. The second embodiment of the present invention is similar to the first embodiment. Therefore, only the differences between the first and second embodiments will be described.

The reference numerals between the first and second embodiments are the same with the addition of 100.

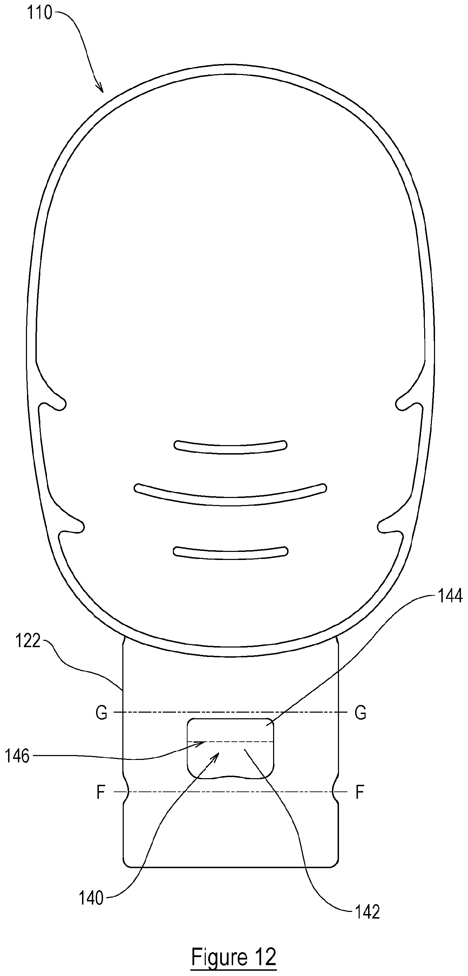

A second embodiment of the invention is shown at figure 12. **In** this embodiment the user graspable member 140 lies entirely within the periphery of the second stiffening member 126b. Such a modification may be advantageous in enabling a user to more readily access and grasp the graspable portion 142 of the user graspable member 140, particularly if they have limited dexterity and/or poor eyesight and so cannot readily see where the user graspable member 140 is.

A third exemplary embodiment of the present invention will now be described.

The third embodiment of the present invention is similar to the first and second embodiments. Therefore, only the differences between the first, second and third embodiments will be described.

The reference numerals between the first and third embodiments are the same with the addition of 200.

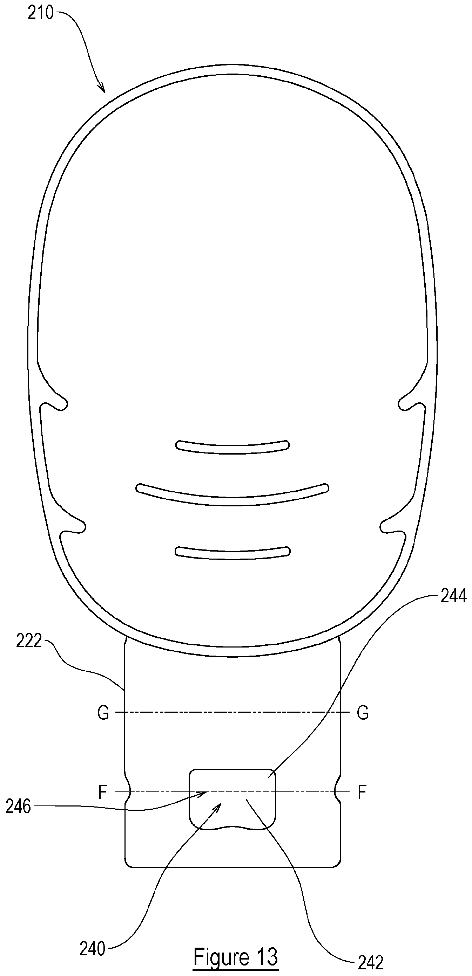

A third embodiment of the invention is shown at figure 13. In this embodiment the user graspable member 240 lies between the first and second stiffening members 226a, 226b. In particular, the connection portion 246 of the user graspable member 240 lies on a fold line F-F defined between the first and second stiffening members 226a, 226b.

When used in this specification and claims, the terms "comprises" and "comprising" and variations thereof mean that the specified features, steps or integers are included. The terms are not to be interpreted to exclude the presence of other features, steps or components.

Although certain example embodiments of the invention have been described, the scope of the appended claims is not intended to be limited solely to these embodiments. The invention is defined by the claims.

## Claims

1. An ostomy appliance (10) including:
first and second walls (12, 14) connected to each other at or near their peripheries;
a stoma-receiving opening (16) for receipt of a stoma;
a collecting cavity (18) defined between the first and second walls wherein the stoma-receiving opening is fluidly connected to the collecting cavity;
a connection member (20) connected to the first and/or second wall for attaching the appliance to a user or for attaching the appliance to a flange for attaching the appliance to a user;
an outlet (22) which extends away from the stoma-receiving opening, the outlet including an outlet wall and terminating in an opening (24); and
**characterised by** a user graspable member (40) for assisting in moving the outlet opening from a first, closed, condition to a second, open, condition;
wherein the user graspable member includes a graspable portion (42) and an attachment portion (44), the attachment portion being positioned above the graspable portion (42) and closer to the stoma-receiving opening than the graspable portion;
wherein the attachment portion is attached to the outlet wall.

2. An ostomy appliance (10) according to claim 1 wherein the user graspable member (40) is positioned at or near the outlet opening (24).

3. An ostomy appliance (10) according to any preceding claim wherein the attachment portion (44) is positioned entirely above the graspable portion (42).

4. An ostomy appliance (10) according to any preceding claim wherein the graspable portion (42) is movable, e.g. pivotally movable, relative to the attachment portion (44); optionally
wherein the graspable portion has a free end (48) remote from the attachment portion which is moveable by a user away from the outlet wall.

5. An ostomy appliance (10) according to any preceding claim wherein the graspable portion (42) is attached to the attachment portion (44) by a connection portion (46); and
wherein the graspable portion is pivotable about the connection portion; optionally
wherein the connection portion defines an axis A about which the graspable portion pivots.

6. An ostomy appliance (10) according to any preceding claim wherein the outlet (22) is foldable between an extended position and a stowed position;
wherein the attachment portion (44) and graspable portion (42) lie in generally the same plane in the stowed position.

7. An ostomy appliance (10) according to any preceding claim including a stiffening member (26a, 26b) attached to or forming part of the outlet wall and positioned at or near the outlet opening (24).

8. An ostomy appliance (10) according to claim 7 wherein the stiffening member (26a, 26b) includes a first edge (30) and a second edge (32), where the stiffening member second edge is positioned closer to the stoma-receiving opening than the stiffening member first edge, and wherein a height Hs of the stiffening member is defined between the first and second stiffening member edges; and
wherein a periphery of the user graspable member (40) includes a first edge (52) and a second edge (54), where the user graspable member second edge is positioned closer to the stoma-receiving opening than the user graspable member first edge, and wherein a height Ht of the user graspable member is defined between the first and second user graspable member edges;
wherein the height Ht is less than the height Hs.

9. An ostomy appliance (10) according to claim 7 or claim 8 wherein the user graspable member (40) lies entirely within a periphery of the stiffening member (26a, 26b).

10. An ostomy appliance (10) according to any preceding claim including a security flap (27) to secure the outlet (22) in a stowed position.

11. An ostomy appliance (10) according to any preceding claim wherein the graspable portion (42) includes a formation (50) for receipt of a user's finger to permit a user to grasp the user graspable member; optionally
wherein the formation forms part of a periphery of the user graspable member; optionally
wherein the formation is a recess of the periphery; optionally
wherein the recess is curved or arcuate; and/or
wherein the recess extends towards the attachment portion (44).

12. An ostomy appliance (10) according to claim 11 wherein the formation (50) provides a different tactile or visual characteristic to a remainder of the graspable portion (42) to indicate to a user which portion of the user graspable member (40) to grasp.

13. An ostomy appliance (10) according to any preceding claim wherein the user graspable member (40) is formed of a synthetic material.

14. An ostomy appliance (10) according to claim 13 wherein the user graspable member (40) is formed of polyester.

15. An ostomy appliance (10) according to any preceding claim wherein the outlet wall is a continuation of or connected to one of the first or second walls (12, 14) which define the collecting cavity (18).

## Patentansprüche

1. Ostomievorrichtung (10), beinhaltend:
eine erste und eine zweite Wand (12, 14), die an oder nahe ihren Peripherien miteinander verbunden sind;
eine Stomaaufnahmeöffnung (16) zur Aufnahme eines Stomas;
einen Sammelhohlraum (18), der zwischen der ersten und zweiten Wand definiert ist, wobei die Stomaaufnahmeöffnung fluidisch mit dem Sammelhohlraum verbunden ist;
ein Verbindungselement (20), das mit der ersten und/oder zweiten Wand verbunden ist, zum Anbringen der Vorrichtung an einem Benutzer oder zum Anbringen der Vorrichtung an einem Flansch zum Anbringen der Vorrichtung an einem Benutzer;
einen Auslass (22), der sich weg von der Stomaaufnahmeöffnung erstreckt, wobei der Auslass eine Auslasswand beinhaltet und in einer Öffnung (24) endet; und
**gekennzeichnet durch** ein von dem Benutzer ergreifbares Element (40) zum Unterstützen beim Bewegen der Auslassöffnung von einem ersten, geschlossenen Zustand zu einem zweiten, offenen Zustand;
wobei das von dem Benutzer ergreifbare Element einen ergreifbaren Abschnitt (42) und einen Anbringungsabschnitt (44) beinhaltet, wobei der Anbringungsabschnitt oberhalb des ergreifbaren Abschnitts (42) und näher an der Stomaaufnahmeöffnung positioniert ist als der ergreifbare Abschnitt;
wobei der Anbringungsabschnitt an der Auslasswand angebracht ist.

2. Ostomievorrichtung (10) nach Anspruch 1, wobei das von dem Benutzer ergreifbare Element (40) an oder nahe der Auslassöffnung (24) positioniert ist.

3. Ostomievorrichtung (10) nach einem vorhergehenden Anspruch, wobei der Anbringungsabschnitt (44) vollständig oberhalb des ergreifbaren Abschnitts (42) positioniert ist.

4. Ostomievorrichtung (10) nach einem vorhergehenden Anspruch, wobei der ergreifbare Abschnitt (42) relativ zu dem Anbringungsabschnitt (44) bewegbar, z. B. schwenkend bewegbar, ist; optional,
wobei der ergreifbare Abschnitt ein freies Ende (48) fern von dem Anbringungsabschnitt aufweist, das, von einem Benutzer, weg von der Auslasswand bewegbar ist.

5. Ostomievorrichtung (10) nach einem vorhergehenden Anspruch, wobei der ergreifbare Abschnitt (42) durch einen Verbindungsabschnitt (46) an dem Anbringungsabschnitt (44) angebracht ist; und
wobei der ergreifbare Abschnitt um den Verbindungsabschnitt schwenkbar ist; optional,
wobei der Verbindungsabschnitt eine Achse A definiert, um die der ergreifbare Abschnitt schwenkt.

6. Ostomievorrichtung (10) nach einem vorhergehenden Anspruch, wobei der Auslass (22) zwischen einer ausgefahrenen Position und einer verstauten Position faltbar ist;
wobei der Anbringungsabschnitt (44) und der ergreifbare Abschnitt (42) in der verstauten Position im Allgemeinen in derselben Ebene liegen.

7. Ostomievorrichtung (10) nach einem vorhergehenden Anspruch, beinhaltend ein Versteifungselement (26a, 26b), das an der Auslasswand angebracht oder Bestandteil davon und an oder nahe der Auslassöffnung (24) positioniert ist.

8. Ostomievorrichtung (10) nach Anspruch 7, wobei das Versteifungselement (26a, 26b) eine erste Kante (30) und eine zweite Kante (32) beinhaltet, wo die zweite Kante des Versteifungselements näher an der Stomaaufnahmeöffnung positioniert ist als die erste Kante des Versteifungselements und wobei eine Höhe Hs des Versteifungselements zwischen den Kanten des ersten und zweiten Versteifungselements definiert ist; und
wobei eine Peripherie des von dem Benutzer ergreifbaren Elements (40) eine erste Kante (52) und eine zweite Kante (54) beinhaltet, wobei die zweite Kante des von dem Benutzer ergreifbaren Elements näher an der Stomaaufnahmeöffnung positioniert ist als die erste Kante des von dem Benutzer ergreifbaren Elements und wobei eine Höhe Ht des von dem Benutzer ergreifbaren Elements zwischen der ersten und der zweiten Kante des von dem Benutzer ergreifbaren Elements definiert ist;
wobei die Höhe Ht weniger ist als die Höhe Hs.

9. Ostomievorrichtung (10) nach Anspruch 7 oder Anspruch 8, wobei das von dem Benutzer ergreifbare Element (40) vollständig innerhalb einer Periphierie des Versteifungselements (26a, 26b) liegt.

10. Ostomievorrichtung (10) nach einem vorhergehenden Anspruch, beinhaltend eine Sicherheitsklappe (27) zum Sichern des Auslasses (22) in einer verstauten Position.

11. Ostomievorrichtung (10) nach einem vorhergehenden Anspruch, wobei der ergreifbare Abschnitt (42) eine Formation (50) zur Aufnahme des Fingers eines Benutzers beinhaltet, um einem Benutzer das Ergreifen des von dem Benutzer ergreifbaren Elements zu gestatten; optional,
wobei die Formation Bestandteil einer Peripherie des von dem Benutzer ergreifbaren Elements ist; optional,
wobei die Formation eine Aussparung der Peripherie ist; optional,
wobei die Aussparung gekrümmt oder bogenförmig ist; und/oder
wobei sich die Aussparung hin zu dem Anbringungsabschnitt (44) erstreckt.

12. Ostomievorrichtung (10) nach Anspruch 11, wobei die Formation (50) einem Rest des ergreifbaren Abschnitts (42) eine unterschiedliche taktile oder visuelle Eigenschaft verleiht, um einem Benutzer anzuzeigen, welcher Abschnitt des von dem Benutzer ergreifbaren Elements (40) zu ergreifen ist.

13. Ostomievorrichtung (10) nach einem vorhergehenden Anspruch, wobei das von dem Benutzer ergreifbare Element (40) aus einem synthetischen Material besteht.

14. Ostomievorrichtung (10) nach Anspruch 13, wobei das von dem Benutzer ergreifbare Element (40) aus Polyester besteht.

15. Ostomievorrichtung (10) nach einem vorhergehenden Anspruch, wobei die Auslasswand eine Fortsetzung einer der ersten oder zweiten Wand (12, 14), wovon der Sammelhohlraum (18) definiert wird, ist oder mit dieser verbunden ist.

## Revendications

1. Appareillage stomique (10), comprenant :
des première et seconde parois (12, 14) reliées l'une à l'autre au niveau ou à proximité de leurs périphéries ;
une ouverture de réception de stomie (16) pour la réception d'une stomie ;
une cavité de collecte (18) définie entre les première et seconde parois, l'ouverture de réception de stomie étant reliée fluidiquement à la cavité de collecte ;
un élément de liaison (20) relié à la première et/ou à la seconde paroi pour fixer l'appareillage à un utilisateur ou pour fixer l'appareillage à une collerette pour fixer l'appareillage à un utilisateur ;
une sortie (22) qui s'étend à l'écart de l'ouverture de réception de stomie, la sortie comprenant une paroi de sortie et se terminant par une ouverture (24) ; et
l'appareillage stomique étant **caractérisé par** un élément saisissable par l'utilisateur (40) pour aider à déplacer l'ouverture de sortie d'un premier état fermé à un second état ouvert ;
l'élément saisissable par l'utilisateur comprenant une partie saisissable (42) et une partie de fixation (44), la partie de fixation étant positionnée au-dessus de la partie saisissable (42) et plus près de l'ouverture de réception de stomie que la partie saisissable ;
la partie de fixation étant fixée à la paroi de sortie.

2. Appareillage stomique (1) selon la revendication 1, l'élément saisissable par l'utilisateur (40) étant positionné au niveau ou à proximité de l'ouverture de sortie (24).

3. Appareillage stomique (10) selon l'une quelconque des revendications précédentes, la partie de fixation (44) étant positionnée entièrement au-dessus de la partie saisissable (42).

4. Appareillage stomique (10) selon l'une quelconque des revendications précédentes, la partie saisissable (42) pouvant être déplacée, par exemple déplacée de manière pivotante, par rapport à la partie de fixation (44) ; éventuellement
la partie saisissable comportant une extrémité libre (48) qui est distante de la partie de fixation et qui peut être déplacée par un utilisateur à l'écart de la paroi de sortie.

5. Appareillage stomique (10) selon l'une quelconque des revendications précédentes, la partie saisissable (42) étant fixée à la partie de fixation (44) par une partie de liaison (46) ; et
la partie saisissable pouvant pivoter autour de la partie de liaison ; éventuellement la partie de liaison définissant un axe A autour duquel la partie saisissable pivote.

6. Appareillage stomique (10) selon l'une quelconque des revendications précédentes, la sortie (22) pouvant être pliée entre une position étendue et une position repliée ;
la partie de fixation (44) et la partie saisissable (42) se situant généralement dans le même plan dans la position repliée.

7. Appareillage stomique (10) selon l'une quelconque des revendications précédentes, comprenant un élément de rigidification (26a, 26b) fixé à la paroi de sortie ou faisant partie de celle-ci, et positionné au niveau ou à proximité de l'ouverture de sortie (24).

8. Appareillage stomique (10) selon la revendication 7, l'élément de rigidification (26a, 26b) comprenant un premier bord (30) et un second bord (32), le second bord de l'élément de rigidification étant positionné plus près de l'ouverture de réception de stomie que le premier bord de l'élément de rigidification, et une hauteur Hs de l'élément de rigidification étant définie entre les premier et second bords de l'élément de rigidification ; et
une périphérie de l'élément saisissable par l'utilisateur (40) comprenant un premier bord (52) et un second bord (54), le second bord de l'élément saisissable par l'utilisateur étant positionné plus près de l'ouverture de réception de stomie que le premier bord de l'élément saisissable par l'utilisateur, et une hauteur Ht de l'élément saisissable par l'utilisateur étant définie entre les premier et second bords de l'élément saisissable par l'utilisateur ;
la hauteur Ht étant inférieure à la hauteur Hs.

9. Appareillage stomique (10) selon la revendication 7 ou 8, l'élément saisissable par l'utilisateur (40) se situant entièrement dans une périphérie de l'élément de rigidification (26a, 26b).

10. Appareillage stomique (10) selon l'une quelconque des revendications précédentes, comprenant un rabat de sécurité (27) pour sécuriser la sortie (22) dans une position repliée.

11. Appareillage stomique (10) selon l'une quelconque des revendications précédentes, la partie saisissable (42) comprenant une formation (50) pour la réception d'un doigt d'un utilisateur pour permettre à un utilisateur de saisir l'élément saisissable par l'utilisateur ; éventuellement
la formation faisant partie d'une périphérie de l'élément saisissable par l'utilisateur ; éventuellement
la formation étant un évidement de la périphérie ; éventuellement
l'évidement étant incurvé ou arqué ; et/ou
l'évidement s'étendant vers la partie de fixation (44).

12. Appareillage stomique (10) selon la revendication 11, la formation (50) conférant une caractéristique tactile ou visuelle différente à un reste de la partie saisissable (42) pour indiquer à un utilisateur quelle partie de l'élément saisissable par l'utilisateur (40) doit être saisie.

13. Appareillage stomique (10) selon l'une quelconque des revendications précédentes, l'élément saisissable par l'utilisateur (40) étant formé d'un matériau synthétique.

14. Appareillage stomique (10) selon la revendication 13, l'élément saisissable par l'utilisateur (40) étant formé de polyester.

15. Appareillage stomique (10) selon l'une quelconque des revendications précédentes, la paroi de sortie étant un prolongement de l'une des première ou seconde parois (12, 14) qui définissent la cavité de collecte (18) ou étant reliée à celle-ci.
